# EUROPEAN PATENT SPECIFICATION

(11) **EP 1 872 824 B1**
(45) Date of publication and mention of the grant of the patent: **26.08.2009**
(21) Application number: 07110880.7
(22) Date of filing: 22.06.2007
(51) Int. Cl.: A61M 39/10

(54) **Connecting structures for luer fitting**
Verbindungsstrukturen für Luer-Anschlüsse
Structures de connexion pour embout Luer

(30) Priority: 28.06.2006 JP 2006178066
(43) Date of publication of application: 02.01.2008
(73) Proprietor: Covidien AG, 8212 Neuhausen am Rheinfall (CH)
(72) Inventor: Ichiro, Kitani, Shizuoka-ken (JP); Norifumi, Fujiwara, Shizuoka-ken (JP); Yosuke, Sakai, Shizuoka-ken (JP)
(74) Representative: Körber, Martin Hans

(56) References cited:
- EP-A- 0 151 519
- US-A- 4 778 447
- US-A1- 2006 033 334
- US-B1- 6 332 633

## Description

### Technical field of the invention

The present invention pertains to a luer-locking fitting connection mechanism for connecting a male connector with a female connector.

### Prior art

Conventionally, the supply of medicinal solutions or blood to a patient, for example, has been conducted using an infusion line or blood transfusion line furnished with a tube. In this case, a luer-locking fitting connection mechanism is used, for example, in order to connect the various tubes that comprise the infusion line. (Refer, for example, to Patent Citation 1.) This type of medical connection apparatus (luer-locking fitting connection mechanism) is furnished with a male connection fitting and a female connection fitting that are connected to the tips of the respective tubes to be connected to each other.

The tip of the male connection fitting is a communicating pipe whose outer wall has a tapered luer shape, with an outer cap structure that surrounds the communicating pipe; the female connection fitting is structured as a tubular body that can be inserted into the outer cap, while the communicating pipe can become engaged within the female connection fitting. Also, a screw thread is formed on the inside wall of the outer cap, and a screw thread is formed on the outside wall of the female connection fitting that engages with the screw thread of the outer cap. Therefore, when the communicating pipe is engaged within the female connection fitting, the screw threads will be screwed together when the outer cap is rotated, thus making it possible to connect the male and female connection fittings together.
Patent Citation 1: Japanese Kokai Patent Application No. Hei 5[1993]-31180.

### Disclosure of the invention

Nevertheless, in the conventional medical connection apparatus described above, if the screw threads are tightened together with too much force, cracks will develop in the female connection fitting, which will cause liquid leakage; if tightened together too loosely, the connection between the male and female connection fittings may come apart, or the fittings may become loose, causing a gap between them that allows the liquid flowing within to leak.

With this situation in mind, the objective of the present invention is to offer a luer-locking fitting connection mechanism that can prevent cracking and liquid leakage.

The objective described above may be accomplished by a luer-locking fitting connection mechanism wherein female threads formed on the internal circumferential surface of a luer-locking fitting member provided with a male connector are caused to be screwed to male threads formed on the external circumferential surface of a female connector, thereby connecting the male connector and the female connector; characterized by the fact that provision is made for an engagement member at the base portion or tip portion of the male threads on the outer surface of the female connector, provision is made for a passive engagement member, at the base portion or tip portion of the female threads on the inner surface of the luer-locking fitting member, that can engage the engagement member, such that when the male threads and the female threads are screwed together in a suitable condition, the engagement member and passive engagement member engage with each other.

In the invented luer-locking fitting connection mechanism having the structure described above, screwing the male threads to the female threads makes it possible to connect the male connector to the female connector; when the screwed-together condition of the male threads and female threads is suitable, the engagement member provided on the outer surface of the female connector and the passive engagement member provided on the inner surface of the luer-locking fitting member will become engaged eliminates. Accordingly, this negates the occurrence of cracks in the female connector or luer-locking fitting member when the male threads and female threads are tightened too strongly, or the leakage of medicinal solutions from a gap between the luer-locking fitting member and female connector when the male threads and female threads are insufficient tightened.

In this case, the suitable screwed-together condition is a condition in which tightening of the male and female threads is suitably strong, neither too tight nor too loose, this being a condition in which leakage does not occur. It is also possible to structure both the engagement member and the passive engagement member as projections, such that these engage when one projection overrides the other projection, or they can be structured with one as a projection and the other as a concavity, such as a groove, for example. Also, the outer surface of the female connector includes outer surfaces other than the external circumferential surface, and the inner surface of the luer-locking fitting member includes inner surfaces other than the internal circumferential surface.

Another structural characteristic of the invented luer-locking fitting connection mechanism is that the engagement member is formed in an approximately annular shape, with an external diameter larger than the male threads, and is provided at the base portion of the male threads on the external circumferential surface of the female connector; the passive engagement member is formed in an approximately annular shape, and is provided at the tip portion of the female threads of the internal circumferential surface of the luer-locking fitting member.

In this case, the base portion of the male threads provided on the female connector is a tube-side portion of the female connector, and the tip portion of the female threads provided on the luer-locking fitting member is the aperture-side portion of the luer-locking fitting member. The passive engagement member can be structured as a concavity or as a projection; if a concavity, the diameter of the passive engagement member comprising the concavity has a size approximately equal to the external diameter of the engagement member, so when the male threads and female threads are screwed together, the male threads will come into contact with the passive engagement member, and the female connector and luer-locking fitting member can be smoothly connected. When the passive engagement member is structured as a projection, when the male threads and female threads are screwed together, the passive engagement member comprising the projection does not come into contact with the male threads of the female connector.

Another structural characteristic of the invented luer-locking fitting connection mechanism is that multiple cut-out portions are provided spaced around the periphery of the tip aperture of the luer-locking fitting member such that the tip aperture can expand outward. This makes it possible to connect the luer-locking fitting member and female connector without damaging the luer-locking fitting member, even if the external diameter of the engagement member is made large.

Another structural characteristic of the invented luer-locking fitting connection mechanism is that the engagement member is formed in an approximately annular shape with an external diameter smaller than the male threads, and this is provided at the tip portion of the male threads on the outer surface of the female connector, while the passive engagement member is formed in an approximately annular shape, and is provided at the base portion of the female threads on the internal surface of the luer-locking fitting member. This also makes it possible to connect the luer-locking fitting member and female connector in a suitable condition. In this case, the engagement member may be provided on the external circumferential surface of the female connector, or it may be provided on the tip surface of the female connector. When the engagement member is provided on the tip surface of the female connector, an end surface or stepwise portion can be formed in the deep interior of the female connector, and the passive engagement member can be provided on the interior end surface or the stepwise portion.

Another structural characteristic of the invented luer-locking fitting connection mechanism is that the main unit of the male connector is structured as a joint part comprising a main joint part to which the luer-locking fitting member is attached and a luer member that can be inserted into the female connector, and the main joint part and the luer member are connected by a joint member that is flexible.

By this means, when the female threads formed on the internal circumferential surface of the luer-locking fitting member are screwed to the male threads formed on the external circumferential surface of the female connector, the luer member, while flexing, will be situated inside the female connector, even if the central axis of the luer member is slightly out of alignment with the central axis of the female connector. Therefore, even if some dimensional error arises between the female connector and luer member, the female connector and male connector will be connected together in a suitable condition. This also makes it difficult for damage to occur. In this case, because the joint member is flexible, it is possible to use a member provided with energizing force, that is expandable and contractible and does not permit the leakage of liquid to the outside, such as a tubular body or bellows-shaped member comprising a soft material, for example.

### Preferred embodiments of the invention

### First embodiment

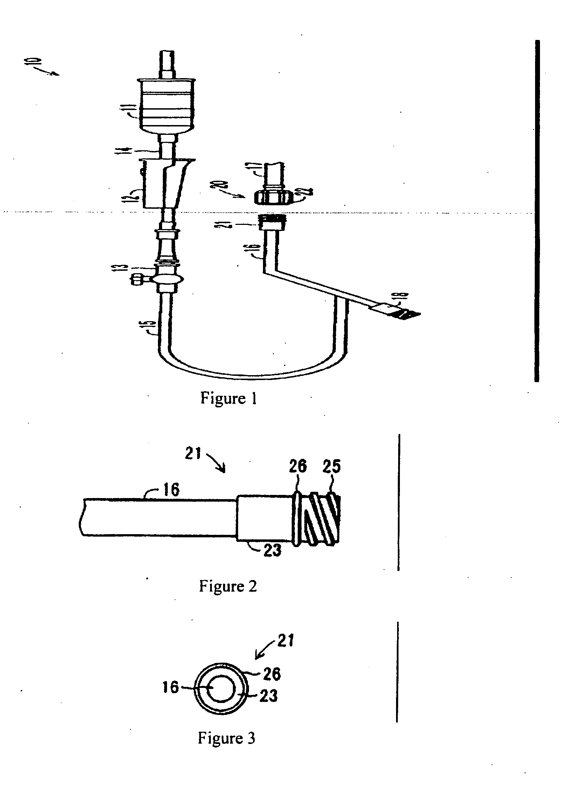

The following statements describe the invention in greater detail using drawings of the luer-locking fitting connection mechanism pertaining to the first embodiment of the invention. Figure 1 shows infusion line set 10 provided with the luer-locking fitting connection mechanism 20 pertaining to this embodiment. The infusion line set 10 is furnished with instillation tube 11, roller clamp 12, and three-way stopcock 13, for example, in order to supply medicinal solutions, for example, to the patient's body. The instillation tube 11 functions internally as a temporary reservoir for the medicinal solutions, and also sends the medicinal solutions to roller clamp 12 at predetermined volume increments via tube 14. The roller clamp 12 is used to adjust the flow rate of medicinal solutions flowing through tube 14.

The three-way stopcock 13 is used to allow medicinal solutions sent from instillation tube 11 to flow downstream or to arrest this flow, and is also used to introduce other medicinal solutions into infusion line set 10. More specifically, three-way stopcock 13 is provided with 3 flow paths: 2 of these flow paths allow the medicinal solutions sent from instillation tube 11 to flow downstream, and the other flow path allows other medicinal solutions to be introduced. The luer-locking fitting connection mechanism 20 is provided downstream of the three-way stopcock 13 via tubes 15 and 16. The luer-locking fitting connection mechanism 20 is structured with a female connector 21 that can be attached to the tip of tube 16, and a male connector 22 that can be attached to tip of tube 17.

As shown in Figures 2-4, the female connector 21 is structured with a tubular main connector unit 23, and a tubular connecting part 24 that extends from the rear tip (base) of main connector unit 23 to the rear. Male threads 25 are provided on the outer circumference of main connector unit 23 from the tip toward the central area, and an annular engagement projection 26 is formed around the circumference at the approximate center, measured axially along the outer circumferential surface of main connector unit 23, at a slight distance from the base of male threads 25. The external diameter of engagement projection 26 is made slightly larger than the external diameter of male threads 25.

The internal circumferential surface 23a of main connector unit 23 is formed in a gently tapered shape such that the diameter on the aperture side is large and gets smaller as it progresses inward. The connecting part 24, having a diameter smaller than the main connector unit 23, extends backward from the back end of internal circumferential surface of main connector unit 23. A gap 24a is formed between the internal circumferential surface of the base of main connector unit 23 and the external circumferential surface in the part of connecting part 24 on the side of the main connector unit 23; by engaging the tip of tube 16 in gap 24a, the female connector 21 can be connected to tube 16. More specifically, connecting part 24 is sandwiched and fixed in a condition wherein the connecting part 24 is inserted inside tube 16, and the base of main connector unit 23 is entered by the tip of tube 16.

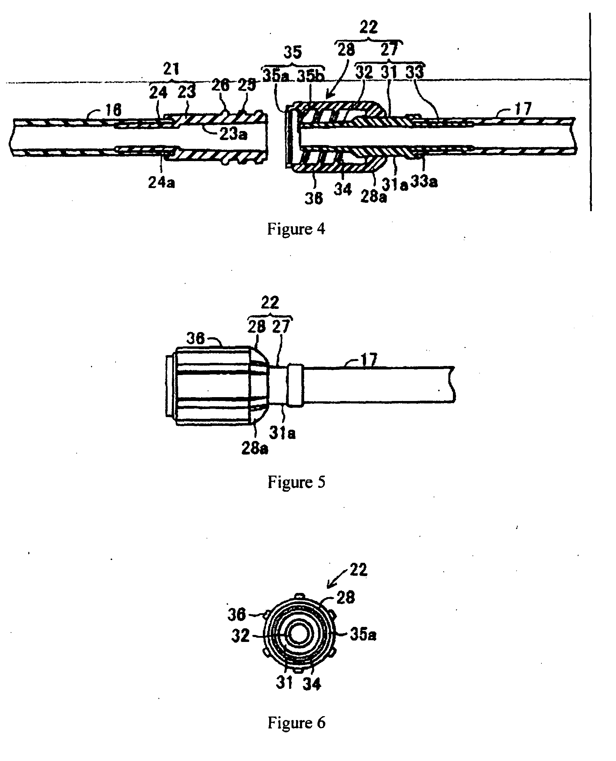

As shown in Figures 4-6, male connector 22 is structured with an approximately tubular joint part 27, and a luer-locking fitting member 28 that is attached to the outer circumference of joint part 27 and can rotate circumferentially about the axis of joint part 27. The joint part 27 is structured with a main joint part 31 formed in a tubular shape, a luer member 32 that extends forward from the tip of main joint part 31, and a tubular connecting part 33 that extends rearward from the base of main joint part 31. The outer circumferential surface of luer member 32 is formed as a gently tapering surface with a large diameter at the base that grows smaller moving toward the tip, such that it can be engaged with the internal circumferential surface 23a of main connector unit 23.

Then, by fitting luer member 32 to main connector unit 23, the female connector 21 and male connector 22 become linked to form a passage. Also, the main joint part 31 is formed with a larger diameter than that of luer member 32, and the portion of main joint part 31 on the same side as luer member 32 is formed with an acutely inclined tapering part. The central portion of main joint part 31, measured axially, is formed so that its external diameter is smaller than that of either end portion, and it is formed with a concavity 31a. The connecting part 33 extends backward from the rear end of main joint part 31. The internal diameter of connecting part 33 is set to be identical to that of main joint part 31 and luer member 32, and the external diameter is set to be approximately the same size as that of luer member 32 (no taper is provided).

Accordingly, the external diameter of connecting part 33 is set to be smaller than the external diameter of main joint part 31. A gap 33a is formed between the internal circumferential surface of the base of main joint part 31 and the external circumferential surface in the part of connecting part 33 on the side of the main joint part 31; by engaging the tip of tube 17 in gap 33a, the male connector 22 may be connected to tube 17. More specifically, connecting part 33 is sandwiched and fixed in a condition where the connecting part 33 is inserted inside tube 17, and the base of main joint part 31 is entered by the tip of tube 17.

The luer-locking fitting member 28 is formed in an approximately cylindrical shape, such that the tip portion of female connector 21 can be housed in its interior; its base 28a has a narrow diameter. More specifically, the size of luer-locking fitting member 28 is provided so that the tip portion of female connector 21 can be placed between it and luer member 32; and the base 28a is engaged in concavity 31a of main joint part 31 such that it can slide in contact with it. Therefore, luer-locking fitting member 28 is able to rotate about the axis of joint part 27, and can move along the axis of joint part 27.

Also, female threads 34 that can screw to the male threads 25 of female connector 21 are formed on the internal circumferential surface of luer-locking fitting member 28, starting from a portion at a predetermined distance from the tip and extending toward the central part, measured axially. Also, a passive engagement member 35 that can engage with engagement projection 26 of female connector 21 is formed at the tip portion of female threads 34 on internal circumferential surface of luer-locking fitting member 28. The passive engagement member 35 is structured with a projecting member 35a provided at the tip of internal circumferential surface of luer-locking fitting member 28, and a groove 35b that is provided between projecting member 35a and the tip of female threads 34.

The engagement projection 26 engages with groove 35b when it overrides projecting member 35a, so that female connector 21 and male connector 22 are connected. Also, the engagement projection 26 and passive engagement member 35 are structured to be engaged when male threads 25 and female threads 34 have been screwed together in a suitable condition (a condition where liquid leakage does not occur, without them being screwed together too strongly); the structure is such that male threads 25 and female threads 34 cannot be screwed together more strongly to engage the engagement projection 26 and the passive engagement member 35.

Also, projecting strips 36 are formed to prevent slippage on the external circumferential surface of luer-locking fitting member 28; they are spaced by a predetermined distance circumferentially and extend axially. Also, tube 15 is connected in a T-shape to the intermediate part of tube 16, and female connector 18 is also provided on the other tip of tube 16. The female connector 18 can also be connected to another male connector, but in the case presented here, female connector 18 is blocked.

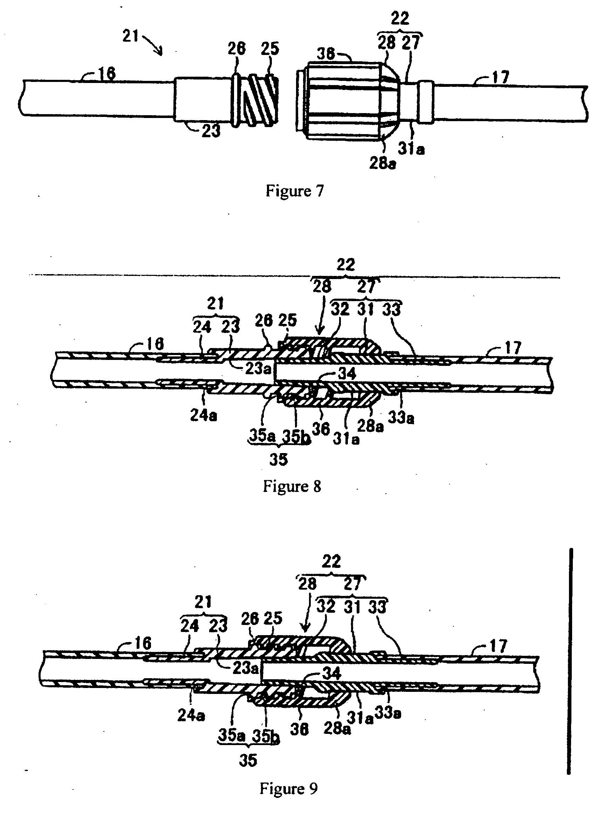

When the female connector 21 and male connector 22 of a luer-locking fitting connection mechanism 20 that has been structured in this way are connected, first, as shown in Figures 4-7, the tip aperture of female connector 21 and the tip aperture of male connector 22 are caused to face each other and are then brought close together. Then the luer member 32 of male connector 22 is placed inside female connector 21, the male threads 25 and female threads 34 are brought into contact, and the luer-locking fitting member 28 is rotated in a predetermined circumferential direction, thus causing the male threads 25 and female threads 34 to be screwed together, as shown in Figure 8.

The luer-locking fitting member 28 is then rotated further such that a suitable screwed-together condition of male threads 25 and female threads 34 is achieved. In this way, as shown in Figure 9, engagement projection 26 of female connector 21 and passive engagement member 35 of male connector 22 become engaged. By this means, female connector 21 and male connector 22 are linked to form a passage in a suitably connected condition that will not give rise to liquid leakage. Also, in this case, the engagement projection 26 of female connector 21 is prevented from retreating backward with respect to male connector 22 by projecting member 35a of passive engagement member 35. Therefore, a suitable screwed-together condition of male threads 25 and female threads 34 is maintained without this screwed-together state of male threads 25 and female threads 34 becoming looser.

Then, when infusion line set 10 is to be used, the downstream end of tube 17 is connected to a puncturing member (not shown in the drawing), such as an indwelling needle, for example, that can be used to puncture the patient's body and be inserted into it. A medicinal solution chemical is caused to flow from instillation tube 11 into the respective tubes 14, 15, 16, and 17, and after the air within tube 14, for example, has been released to the outside, roller clamp 12 is adjusted and the flow of medicinal solution is caused to stop. In this condition, puncturing member is inserted into a predetermined location in the patient's body, and roller clamp 12 is adjusted such that the flow rate of medicinal solution flowing within tube 14, for example, is adjusted to a suitable amount. By this means, the predetermined amount of medicinal solution is supplied from instillation tube 11 into the patient's body.

In this way, the luer-locking fitting connection mechanism 20 of the present embodiment is structured so that when the screwed-together condition of male threads 25 and female threads 34 is suitably formed to connect female connector 21 and male connector 22, the engagement projection 26 provided on the external circumferential surface of female connector 21 is engaged with the passive engagement member 35 provided to the internal circumferential surface of luer-locking fitting member 28. Accordingly, the male threads 25 and female threads 34 will be strongly tightened to eliminate problems such as the formation of cracks in female connector 21 or luer-locking fitting member 28, the leakage of medicinal solution if a gap is formed between female connector 21 and luer-locking fitting member 28, and loosening between male threads 25 and female threads 34.

### Second embodiment

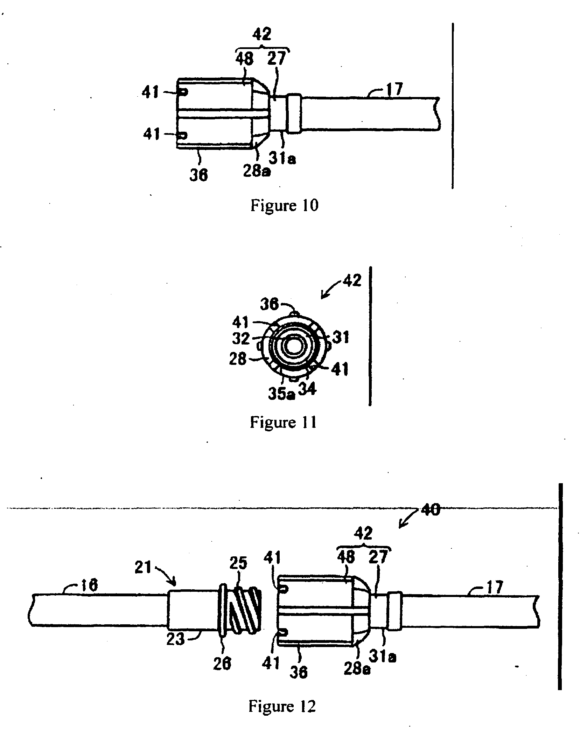

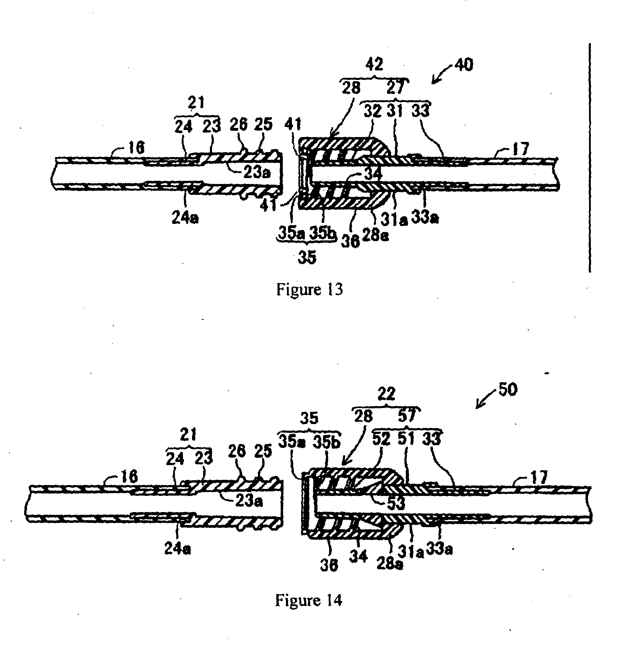

Figures 10 and 11 show a male connector 42 provided with the luer-locking fitting connection mechanism 40 (refer to Figures 12 and 13) pertaining to the second embodiment of the invention. The male connector 42 is formed with 4 similarly shaped cut-out portions 41 that are separated by a predetermined distance around the circumference of the aperture rim of luer-locking fitting member 48. Each of the cut-out portions 41 passes completely through luer-locking fitting member 48 from its inner surface to its outer surface, and as shown in Figure 13, they extend from the aperture rim of luer-locking fitting member 48 to the part where passive engagement member 45 is formed.

By providing cut-out portions 41, it becomes possible for the aperture portion of luer-locking fitting member 48 to expand outward. More specifically, this embodiment has been structured such that, because luer-locking fitting member 48 has elastic properties, when force is applied to enlarge the diameter of the aperture portion, the aperture portion will widen according to the force. The structure of the other elements of luer-locking fitting connection mechanism 40 provided with male connector 42 are identical to the luer-locking fitting connection mechanism 20 described previously. Accordingly, identical parts are indicated by identical keys, and the explanations are omitted.

Due to this structure, even if the external diameter of engagement projection 26 is made slightly large, it is still possible to connect luer-locking fitting member 48 to female connector 21 without damaging luer-locking fitting member 48. In other respects, the operation and effect of luer-locking fitting connection mechanism 40 are identical to the operation and effect of luer-locking fitting connection mechanism 20.

### Third embodiment

Although it is not shown in the drawings, a third embodiment of the invention enables the engagement member to be formed as an approximately annular form, with an external diameter smaller than the male threads, that does not come into contact with the female threads of the luer-locking fitting member and is provided at the tip of the female connector, while the passive engagement member is formed as an approximately annular concavity provided at the interior end of the luer-locking fitting member. This arrangement also permits the male connector and female connector to be connected together in a suitable condition.

### Fourth embodiment

Figure 14 shows luer-locking fitting connection mechanism 50 pertaining to the fourth embodiment of the invention. In this luer-locking fitting connection mechanism 50, the main joint part 51 and luer member 52 that form the joint part 57 are connected by a tubular joint member 53 that is provided with flexibility and elongation properties. The structure of the other elements of luer-locking fitting connection mechanism 50 are identical to the luer-locking fitting connection mechanism 20 described previously. Accordingly, identical parts are indicated by identical keys, and the explanations are omitted.

Due to this structure, when the female threads 34 of luer-locking fitting member 28 are screwed to male threads 25 of female connector 21, the luer member 52, while flexing, will be situated inside female connector 21 even if the central axis of luer member 52 is slightly out of alignment with the central axis of female connector 21. Therefore, even if some dimensional error arises between female connector 21 and luer member 52, the female connector 21 and male connector 22 will be connected together in a suitable condition. Also, even if there is some dimensional discrepancy longitudinally between the female connector 21 and male connector 22, the female connector 21 and male connector 22 will be connected together in a suitable condition because of joint member 53. This also makes it difficult for damage to occur to luer member 52.

Furthermore, the invented luer-locking fitting connection mechanism is not limited to the embodiments described above, but may be realized by making suitable changes. For example, in the embodiment described above, the luer-locking fitting connection mechanism was furnished with infusion line set 10 provided with an instillation tube 11, but it is also possible to use the luer-locking fitting connection mechanism to connect to a blood transfusion line or a tube member used for some other purpose, instead of to an infusion line.

Also, in the embodiments described above, the passive engagement member is structured as a projecting member and a groove, but the engagement member may also be structured as only a projecting member or a groove alone. When the passive engagement member is structured as only a projecting member, the design may be such that the engagement member and the passive engagement member become engaged when the engagement member overrides the passive engagement member, or when the engagement member comes into contact with the passive engagement member, the passive engagement member acts as a stop, such that the engagement member and passive engagement member engage with each other. Also, the engagement member and/or passive engagement member are not limited to having annular shapes; they may also be structured as concavities or projections that are short in the lengthwise dimension. The structure of the other parts of the invented luer-locking fitting connection mechanism may also be changed suitably within the technical scope of the present invention.

### Brief description of the figures

Figure 1: a block diagram showing an infusion line set provided with the luer-locking fitting connection mechanism pertaining to the first embodiment of the invention.
Figure 2: a side view showing the female connector.
Figure 3: a rear view showing the female connector.
Figure 4: a cross section showing the luer-locking fitting connection mechanism prior to connection of the female connector to the male connector.
Figure 5: a side view showing the male connector.
Figure 6: a front view showing the male connector.
Figure 7: a side view of the luer-locking fitting connection mechanism prior to connection of the female connector to the male connector.
Figure 8: a cross section showing the condition wherein the female connector and male connector are connected.
Figure 9: a cross section showing the condition wherein the female connector and male connector are connected.
Figure 10: a side view showing the male connector furnished with the luer-locking fitting connection mechanism pertaining to the second embodiment of the invention.
Figure 11: a front view of the male connector shown in Figure 10.
Figure 12: a side view of the luer-locking fitting connection mechanism prior to connection of the female connector to the male connector.
Figure 13: a cross section showing the luer-locking fitting connection mechanism prior to connection of the female connector to the male connector.
Figure 14: cross section showing the luer-locking fitting connection mechanism pertaining to the fourth embodiment of the invention.

### Explanation of symbols

- 20, 40, 50: Luer-locking fitting connection mechanism
- 21: Female connector
- 22, 42: Male connector
- 25: Male threads
- 26: Engagement projection
- 27: Joint part
- 28, 48: Luer-locking fitting member
- 34: Female threads
- 35,45: Passive engagement member
- 35a: Projecting member
- 35b: Groove
- 41: Cut-out portion
- 51: Main joint part
- 52: Luer member
- 53: Joint member

## Claims

1. A luer-locking fitting connection mechanism wherein female threads formed on the internal circumferential surface of a luer-locking fitting member provided with a male connector is caused to be screwed together with male threads formed on the external circumferential surface of a female connector, thereby connecting the aforementioned male connector and the aforementioned female connector;
**characterized by** the fact that provision is made for an engagement member at the base portion or tip portion of the aforementioned male threads on the outer surface of the aforementioned female connector; provision is made for a passive engagement member, at the base portion or tip portion of the aforementioned female threads on the inner surface of the aforementioned luer-locking fitting member, that can engage the aforementioned engagement member; such that when the aforementioned male threads and female threads are screwed together in a suitable condition, the aforementioned engagement member and passive engagement member engage with each other.

2. The luer-locking fitting connection mechanism according to Claim 1, wherein the aforementioned engagement member is formed in an approximately annular shape, with an external diameter larger than the aforementioned male threads, and is provided on the base portion of the aforementioned male threads on the external circumferential surface of the aforementioned female connector; and the aforementioned passive engagement member is formed in an approximately annular shape, and is provided on the tip portion of the aforementioned female threads on the internal circumferential surface of the aforementioned luer-locking fitting member.

3. The luer-locking fitting connection mechanism according to Claim 2, wherein multiple cut-out portions are provided spaced around the periphery of the tip aperture of the aforementioned luer-locking fitting member such that the aforementioned tip aperture can expand outward.

4. The luer-locking fitting connection mechanism according to Claim 1, wherein the aforementioned engagement member is formed in an approximately annular shape with an external diameter smaller than the aforementioned male threads, and is provided on the tip portion of the aforementioned male threads on the outer surface of the aforementioned female connector, and the aforementioned passive engagement member is formed in an approximately annular shape, and is provided on the base portion of the aforementioned female threads on the internal surface of the aforementioned luer-locking fitting member.

5. The luer-locking fitting connection mechanism according to any of Claims 1-4, wherein the main unit of the aforementioned male connector is structured with a joint part comprising a main joint part to which the aforementioned luer-locking fitting member is attached and a luer member that can be inserted into the aforementioned female connector, and the aforementioned main joint part and the aforementioned luer member are connected by a joint member that has flexible properties.

## Patentansprüche

1. Verbindungsmechanismus für einen Luer-Lock-Anschluss, wobei die Innengewinde, die an der Innenumfangsfläche eines mit einem männlichen Anschluss versehenen Luer-Lock-Anschlusselements ausgebildet sind, dazu dienen, mit den an der Außenumfangsfläche eines weiblichen Anschlusses ausgebildeten Außengewinden zusammengeschraubt zu werden, wodurch der oben erwähnte männliche Anschluss mit dem oben erwähnten weiblichen Anschluss verbunden wird;
durch die Tatsache **gekennzeichnet**, dass ein Eingriffselement an dem Endstück oder Spitzenstück der oben erwähnten Außengewinde an der Außenseite des oben erwähnten weiblichen Anschlusses vorgesehen ist; dass ein passives Eingriffselement an dem Endstück oder Spitzenstück der oben erwähnten Innengewinde an der Innenseite des oben erwähnten Luer-Lock-Anschlusselements vorgesehen ist, in das das oben erwähnte Eingriffselement eingreifen kann; so dass, wenn die oben erwähnten Außengewinde und Innengewinde in einer geeigneten Stellung zusammengeschraubt sind, das oben erwähnte Eingriffselement und das passive Eingriffselement ineinander eingreifen.

2. Verbindungsmechanismus für einen Luer-Lock-Anschluss gemäß Anspruch 1, wobei das oben erwähnte Eingriffselement in einer annähernd ringförmigen Form ausgebildet ist, wobei der Außendurchmesser größer ist als die oben erwähnten Außengewinde, und auf dem Endstück der oben erwähnten Außengewinde an der Außenumfangsfläche des oben erwähnten weiblichen Anschlusses vorgesehen ist; und wobei das oben erwähnte passive Eingriffselement in einer annähernd ringförmigen Form ausgebildet ist, und auf dem Spitzenstück der oben erwähnten Innengewinde an der Innenumfangsfläche des oben erwähnten Luer-Lock-Anschlusselements vorgesehen ist.

3. Verbindungsmechanismus für einen Luer-Lock-Anschluss gemäß Anspruch 2, wobei mehrere ausgeschnittene Teile um den Umfang der Spitzenöffnung des oben erwähnten Luer-Lock-Anschlusselements beabstandet vorgesehen sind, so dass die oben erwähnte Spitzenöffnung sich nach außen erweitern kann.

4. Verbindungsmechanismus für einen Luer-Lock-Anschluss gemäß Anspruch 1, wobei das oben erwähnte Eingriffselement in einer annähernd ringförmigen Form mit einem kleineren Außendurchmesser als die oben erwähnten Außengewinde ausgebildet ist, und auf dem Spitzenstück der oben erwähnten Außengewinde an der Außenfläche des oben erwähnten weiblichen Anschlusses vorgesehen ist; und das oben erwähnte passive Eingriffselement in einer annähernd ringförmigen Form ausgebildet ist und auf dem Endstück der oben erwähnten Innengewinde an der Innenfläche des oben erwähnten Luer-Locking-Anschlusselements vorgesehen ist.

5. Verbindungsmechanismus für einen Luer-Lock-Anschluss gemäß einem der Ansprüche 1-4, wobei die Haupteinheit des oben erwähnten männlichen Anschlusses mit einem Gelenkteil gestaltet ist, das ein Hauptgelenkteil aufweist, an dem das oben erwähnte Luer-Lock-Anschlusselement angebracht ist, und ein Luer-Element, dass in den oben erwähnten weiblichen Anschluss eingesteckt werden kann, und wobei das oben erwähnte Hauptgelenkteil und das oben erwähnte Luer-Element durch ein Gelenkelement verbunden sind, das flexible Eigenschaften besitzt.

## Revendications

1. Mécanisme de connexion pour embout Luer, où des filets femelles formés sur la surface circonférentielle intérieure d'un élément d'embout Luer muni d'un connecteur mâle est amené à être vissé ensemble avec les filets males formés sur la surface circonférentielle externe d'un connecteur femelle, en connectant ainsi le connecteur mâle précité et le connecteur femelle précité;
**caractérisé par le fait qu'**il est réalisé un élément d'engagement à la portion de base ou portion de pointe des filets mâles précités sur la surface externe du connecteur femelle précité; qu'il est réalisé un élément d'engagement passif, à la portion de base ou la portion de pointe des filets femelles précités sur la surface interne de l'élément d'embout Luer précité, qui peut être mis en prise avec l'élément d'engagement précité de sorte que lorsque les filets mâles et les filets femelles précités sont vissés ensemble dans un état approprié, l'élément d'engagement précité et l'élément d'engagement passif viennent en prise l'un avec l'autre.

2. Mécanisme de connexion pour embout Luer selon la revendication 1, où l'élément d'engagement précité est réalisé en une forme approximativement annulaire, avec un diamètre externe plus grand que les filets mâles précités, et est réalisé sur la portion de base des filets mâles précités sur la surface circonférentielle externe du connecteur femelle précité; et l'élément d'engagement passif précité est réalisé en une forme approximativement annulaire et est réalisé sur la portion de pointe des filets femelles précités sur la surface circonférentielle intérieure de l'élément pour embout Luer précité.

3. Mécanisme de connexion pour embout Luer selon la revendication 2, où des portions découpées multiples sont espacées autour de la périphérie de l'ouverture de pointe de l'élément d'embout Luer précité de sorte que l'ouverture de pointe précitée peut s'expanser vers l'extérieur.

4. Mécanisme de connexion pour embout Luer selon la revendication 1, où l'élément d'engagement précité est réalisé en une forme approximativement annulaire, avec un diamètre externe plus petit que les filets mâles précités et est réalisé sur la portion de pointe des filets mâles précités sur la surface extérieure du connecteur femelle précité, et l'élément d'engagement passif précité est réalisé en une forme approximativement annulaire, et est réalisé sur la portion de base des filets femelles précités sur la surface interne de l'élément pour embout Luer précité.

5. Mécanisme de connexion pour embout Luer selon l'une quelconque des revendications 1 à 4, où l'unité principale du connecteur mâle précité est structurée avec une partie de jonction comprenant une partie de jonction principale à laquelle l'élément d'embout Luer précité est fixé, et un élément Luer qui peut être inséré dans le connecteur femelle précité, et la partie de jonction principale précitée et l'élément de Luer précité sont connectés par un élément de jonction qui a des propriétés flexibles.
